# EUROPEAN PATENT APPLICATION

(11) **EP 4 620 419 A1**
(43) Date of publication of application: **24.09.2025**
(21) Application number: 25161900.3
(22) Date of filing: 05.03.2025
(51) Int. Cl.: A61B 34/20, G06T 7/00, A61B 1/00

(54) **INFORMATION PROCESSING APPARATUS AND INFORMATION PROCESSING PROGRAM**

(30) Priority: 18.03.2024 JP 2024042841
(71) Applicant: FUJI-FILM Corporation, Minato-ku Tokyo 106-8620 (JP)
(72) Inventor: CHIBA, Haruto, Tokyo (JP)
(74) Representative: Dehns Germany Partnerschaft mbB

(57) **Abstract**

In a case where an endoscopic image, from which a viewpoint of an endoscope is not trackable, is obtained, the viewpoint of the endoscope is prevented from being estimated from the endoscopic image without any change, and information is prevented from being notified based on erroneous estimation.

An information processing apparatus (3) acquires an endoscopic image (4) and estimates whether or not a viewpoint of an endoscope (2) that has captured the endoscopic image (4) is trackable from the endoscopic image (4). In a case where it is estimated that the viewpoint of the endoscope (2) is not trackable, the information processing apparatus (3) temporarily stops tracking of the viewpoint of the endoscope (2) until the endoscopic image (4), from which it is estimated that the viewpoint of the endoscope (2) is trackable, is acquired.

## Description

### BACKGROUND OF THE INVENTION

### 1. Field of the Invention

The present invention relates to an information processing apparatus and an information processing program.

### 2. Description of the Related Art

For example, WO2014/141968A discloses an endoscope system that estimates a position of a distal end of an endoscope using image matching between an endoscopic image and a virtual endoscopic image generated from three-dimensional image data.

WO2007/129493A discloses a medical image observation support device that generates a route of a luminal organ to reach a target part set for three-dimensional image data and determines which branch tube in a bifurcation portion the endoscope is advanced to in a case where the endoscope reaches the bifurcation portion.

WO2016/009701A discloses a navigation system that determines whether or not a position of a bifurcation tube is closer to a distal end than a current position of a viewpoint in a case where it is determined that the target bifurcation tube has not been observed and waits until the target bifurcation tube is observed in a case where it is determined that the target bifurcation tube is closer to the distal end.

### SUMMARY OF THE INVENTION

In endoscopy or the like, an information processing apparatus may be used that estimates a viewpoint of an endoscope in a living body from an endoscopic image and notifies a medical worker of a traveling direction of the endoscope such that the endoscope reaches a target location.

For example, in a case where an unknown structure region, which is a region that makes it difficult to specify the shape or pattern of an object, is included in the endoscopic image, information obtained from the endoscopic image may be reduced, and the accuracy of estimation of the viewpoint of the endoscope may be reduced, as compared to a case where the unknown structure region is absent.

In this case, in a case where the traveling direction of the endoscope is determined using the estimated viewpoint of the endoscope without any change, a situation in which the medical worker is notified of an incorrect traveling direction may occur.

The present disclosure has been made in view of the above circumstances, and an object of the present disclosure is to provide an information processing apparatus and an information processing program that, in a case where an endoscopic image, from which a viewpoint of an endoscope is not trackable, is obtained, prevent the viewpoint of the endoscope from being estimated from the endoscopic image without any change and information based on erroneous estimation from being notified.

According to a first aspect of the technology of the present disclosure, there is provided an information processing apparatus comprising: an acquisition unit that acquires an endoscopic image of a living body; an estimation unit that estimates whether or not a viewpoint of an endoscope that has captured the endoscopic image is trackable from the endoscopic image acquired by the acquisition unit; and a controller that, in a case where the estimation unit estimates that the viewpoint of the endoscope is not trackable, performs control to temporarily stop tracking of the viewpoint of the endoscope until the acquisition unit acquires the endoscopic image from which the estimation unit estimates that the viewpoint of the endoscope is trackable.

According to a first embodiment of the technology of the present disclosure, in the information processing apparatus according to the first aspect, the estimation unit may estimate whether or not the viewpoint of the endoscope that has captured the endoscopic image is trackable, based on a score indicating a degree of trackability of the viewpoint of the endoscope obtained from the endoscopic image.

According to a second embodiment of the technology of the present disclosure, in the information processing apparatus according to the first aspect, the estimation unit may estimate whether or not the viewpoint of the endoscope that has captured the endoscopic image is trackable, based on a classification result of classifying the endoscopic image into any of a plurality of classes including a trackable class and an untrackable class.

According to a third embodiment of the technology of the present disclosure, in the information processing apparatus according to the first aspect, the estimation unit may estimate whether or not the viewpoint of the endoscope that has captured the endoscopic image is trackable, using a type of an unknown structure region that is included in the endoscopic image and that is determined in advance to affect the tracking of the viewpoint of the endoscope.

According to a fourth embodiment of the technology of the present disclosure, in the information processing apparatus according to the first aspect, the estimation unit may estimate whether or not the viewpoint of the endoscope that has captured the endoscopic image is trackable, using a range of an unknown structure region that is included in the endoscopic image and that is determined in advance to affect the tracking of the viewpoint of the endoscope.

According to a fifth embodiment of the technology of the present disclosure, in the information processing apparatus according to any one of the first aspect or first to fourth embodiments, the estimation unit may estimate whether or not the viewpoint of the endoscope is trackable before the controller tracks the viewpoint of the endoscope.

According to a sixth embodiment of the technology of the present disclosure, in the information processing apparatus according to any one of the first aspect or first to fourth embodiments, the estimation unit may estimate whether or not the viewpoint of the endoscope is trackable after the controller tracks the viewpoint of the endoscope.

According to a seventh embodiment of the technology of the present disclosure, in the information processing apparatus according to any one of the first aspect or first to fourth embodiments, the controller may control the acquisition unit such that the acquisition unit continues to acquire the endoscopic image and may control the estimation unit such that the estimation unit continues to estimate whether or not the viewpoint of the endoscope is trackable from the endoscopic image acquired by the acquisition unit even during a period for which the tracking of the viewpoint of the endoscope is temporarily stopped.

According to an eighth embodiment of the technology of the present disclosure, in the information processing apparatus according to the seventh embodiment, the controller may perform control to resume the tracking of the viewpoint of the endoscope in a case where the estimation unit estimates that the viewpoint of the endoscope is trackable for a new endoscopic image acquired by the acquisition unit during the period for which the tracking of the viewpoint of the endoscope is temporarily stopped.

According to a second aspect of the technology of the present disclosure, there is provided an information processing program causing a computer to execute a process comprising: acquiring an endoscopic image of a living body; estimating whether or not a viewpoint of an endoscope that has captured the endoscopic image is trackable from the acquired endoscopic image; and in a case where it is estimated that the viewpoint of the endoscope is not trackable, temporarily stopping tracking of the viewpoint of the endoscope until the endoscopic image, from which it is estimated that the viewpoint of the endoscope is trackable, is acquired.

According to the present disclosure, in a case where an endoscopic image, from which a viewpoint of an endoscope is not trackable, is obtained, it is possible to prevent the viewpoint of the endoscope from being estimated from the endoscopic image without any change and information based on erroneous estimation from being notified.

### BRIEF DESCRIPTION OF THE DRAWINGS

Fig. 1 is a diagram showing an example of a configuration of an information processing system.
Fig. 2 is a diagram showing an example of an endoscopic image.
Fig. 3 is a diagram showing an example of an endoscopic image including an unknown structure region.
Fig. 4 is a diagram showing an example of machine learning of a trackability estimation model.
Fig. 5 is a diagram showing an example of a bronchial model.
Fig. 6 is a diagram showing an example of a virtual endoscopic image.
Fig. 7 is a diagram showing an example of a method for estimating a viewpoint difference between images.
Fig. 8 is a diagram showing an example of tracking of a viewpoint of an endoscope.
Fig. 9 is a diagram showing an example of machine learning of a viewpoint difference estimation model.
Fig. 10 is a diagram showing an example of a configuration of an information processing apparatus configured by a computer.
Fig. 11 is a flowchart showing an example of a flow of a tracking process according to a first embodiment.
Fig. 12 is a flowchart showing a modification example of the flow of the tracking process according to the first embodiment.
Fig. 13 is a diagram showing another example of the machine learning of a trackability estimation model.
Fig. 14 is a diagram showing an example of machine learning of an unknown structure region discrimination model.
Fig. 15 is a diagram showing an example of machine learning of an unknown structure region detection model.
Fig. 16 is a diagram showing an example of a method for estimating a similarity between images.
Fig. 17 is a flowchart showing an example of a flow of a tracking process according to a second embodiment.
Fig. 18 is a diagram showing an example of estimation of a viewpoint difference of the endoscope using added supplementary information.

### DESCRIPTION OF THE PREFERRED EMBODIMENTS

Hereinafter, embodiments of the present disclosure will be described in detail with reference to the drawings. In addition, the same components and the same processes are denoted by the same reference numerals throughout the drawings, and a duplicate description thereof will be omitted. Dimensional ratios in the drawings are exaggerated for convenience of description and may be different from the actual ratios.

### First Embodiment

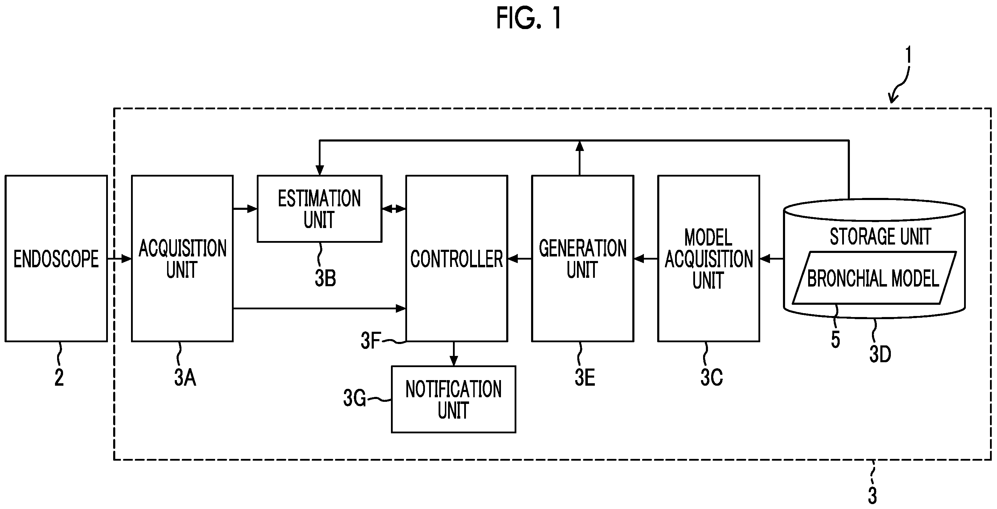

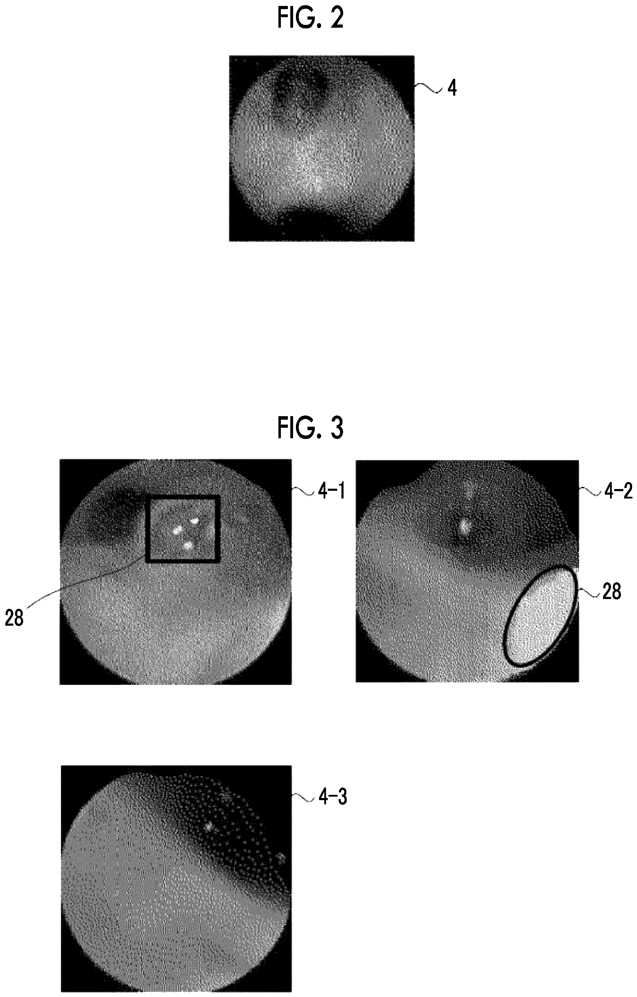

Fig. 1 is a diagram showing an example of a configuration of an information processing system 1 according to a first embodiment. As shown in Fig. 1, the information processing system 1 includes an endoscope 2 and an information processing apparatus 3. An imaging device, such as a camera, is provided at a distal end of the endoscope 2. The information processing system 1 transmits an image captured by the endoscope 2, that is, an endoscopic image 4 to the information processing apparatus 3. Fig. 2 is a diagram showing an example of the endoscopic image 4 captured by the endoscope 2.

There is no restriction on a location of a living body into which the endoscope 2 is inserted, and the endoscope 2 may be, for example, an endoscope 2 for a digestive organ or a ureteroscope. However, as an example, the endoscope 2 according to the embodiment of the present disclosure will be described as a bronchoscope that is inserted into a bronchus of a subject.

In a case where the endoscope 2 is inserted into the bronchus and advanced to a position to be examined, the bronchi have many bifurcations and have a similar shape. Therefore, in some cases, a navigation device is used that notifies a medical worker who operates the endoscope 2 of support information indicating the position and direction of the endoscope 2 in the bronchus and a direction in which the endoscope 2 is to be advanced to reach the position to be examined (hereinafter, referred to as a "target location").

The information processing apparatus 3 is an example of the navigation device that notifies the medical worker of a traveling direction of the endoscope 2. The information processing apparatus 3 includes each functional unit of an acquisition unit 3A, an estimation unit 3B, a model acquisition unit 3C, a storage unit 3D, a generation unit 3E, a controller 3F, and a notification unit 3G.

The acquisition unit 3A acquires the endoscopic image 4 from the endoscope 2.

An image of the inside of the bronchus of the subject is displayed in the endoscopic image 4. However, the endoscopic image 4 may include an unknown structure region. The unknown structure region means a region of the endoscopic image 4 that is not capable of being used to estimate the position and posture of the endoscope 2 in a case where the information processing apparatus 3 estimates the position and posture of the endoscope 2 using the endoscopic image 4.

Examples of the unknown structure region include a region having bubbles attached thereto, a region in which the endoscope 2 is out of focus, a region in which the image is blocked by a substance attached to a lens of the endoscope 2, a reflection region in which light illumination is reflected, a whiteout region, and a blackout region. That is, the unknown structure region is a general term for a region that makes it difficult to specify an object.

Fig. 3 is a diagram showing an example of the endoscopic image 4 including the unknown structure region. In a region indicated by a frame 28 of an endoscopic image 4-1 shown in Fig. 3, bubbles attached to the bronchus which is an example of the unknown structure region are recognized. In a region indicated by a frame 28 of an endoscopic image 4-2, the reflection of illumination light is recognized. Further, an endoscopic image 4-3 is an example of a so-called out-of-focus image in which the endoscope 2 is too close to the bronchus and does not focus on the bronchus.

In a case where the information processing apparatus 3 estimates the position and posture of the endoscope 2 using the endoscopic image 4 including the unknown structure region that does not correctly represent a pattern of a bronchial wall (referred to as a "texture of a bronchus") due to the shape of the bronchus, the state of blood vessels and tissues, and the like, there is a concern that the accuracy of estimation will be reduced, as compared to a case where the endoscopic image 4 in which the unknown structure region is not present is used.

Therefore, the estimation unit 3B estimates whether or not it is possible to track the position and posture of the endoscope 2 which has captured the endoscopic image 4 from the endoscopic image 4 based on attribute information of the unknown structure region, such as the presence or absence, type, and range of the unknown structure region included in the endoscopic image 4. That is, in a case where the estimation unit 3B estimates the position and posture of the endoscope 2 from the endoscopic image 4, the estimation unit 3B estimates whether or not an error of the estimated position and posture of the endoscope 2 with respect to the actual position and posture of the endoscope 2 is included within an allowable range.

In addition, the posture of the endoscope 2 is a direction in which the endoscope 2 is facing. Since an imaging range of the endoscopic image 4 captured by the endoscope 2 is determined by the position and posture of the endoscope 2, hereinafter, the position and posture of the endoscope 2 is referred to as a "viewpoint of the endoscope 2". In addition, whether or not the viewpoint of the endoscope 2 can be tracked is represented using the expression "trackability of the endoscope 2".

The trackability of the viewpoint of the endoscope 2 is determined by, for example, a score that is output from a trackability estimation model 21 in a case where the endoscopic image 4 is input to the trackability estimation model 21. The score is a value representing the degree of trackability of the endoscope 2. For example, as the score is higher, the probability that the viewpoint of the endoscope 2 will be tracked from the input endoscopic image 4 is higher. In the present disclosure, for example, it is assumed that the score represents the probability that the viewpoint of the endoscope 2 will be tracked.

The trackability estimation model 21 is, for example, a model generated in advance by machine learning and is stored in the storage unit 3D in advance. The information processing apparatus 3 shown in Fig. 1 includes the storage unit 3D. However, the information processing apparatus 3 does not necessarily include the storage unit 3D. For example, an external apparatus other than the information processing apparatus 3, such as a data server, may be used as the storage unit 3D.

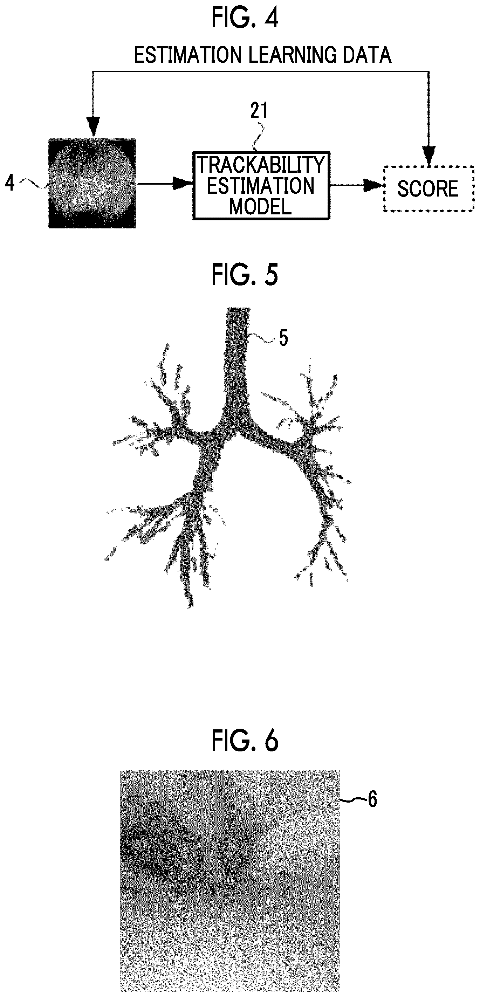

Fig. 4 is a diagram showing an example of the machine learning of the trackability estimation model 21. For example, the medical worker prepares a plurality of learning data items (hereinafter, referred to as "estimation learning data items") in advance in which the score is associated with each of the endoscopic images 4 captured in advance. The medical worker associates the score with each endoscopic image 4 based on the state of the unknown structure region, such as the presence or absence, type, and range of the unknown structure region in the endoscopic image 4.

In addition, in a case where the endoscopic image 4 included in the estimation learning data is input, machine learning is performed on the trackability estimation model 21 such that the score associated with the input endoscopic image 4 is output from the trackability estimation model 21. That is, in the estimation learning data, the endoscopic image 4 is input data, and the score is training data.

For example, a convolutional neural network (CNN) or the like is used as the trackability estimation model 21. For example, deep learning is used as a machine learning method.

The estimation unit 3B notifies the controller 3F of the score obtained from the trackability estimation model 21.

In Fig. 1, the controller 3F that has received the score from the estimation unit 3B compares the received score with a predetermined reference score. The reference score is a value indicating a minimum score at which an estimation error of the viewpoint of the endoscope 2 is considered to be included within an allowable range in a case where the viewpoint of the endoscope 2 is tracked, is set in advance by, for example, the medical worker, and is stored in the storage unit 3D in advance.

In a case where the received score is less than the reference score, there is a high probability that the tracked viewpoint of the endoscope 2 will be incorrect even though the viewpoint of the endoscope 2 is tracked from the endoscopic image 4 acquired by the acquisition unit 3A. Therefore, the controller 3F performs control to temporarily stop the tracking of the viewpoint of the endoscope 2 until a score equal to or greater than the reference score is newly received from the estimation unit 3B, that is, until the acquisition unit 3A acquires the endoscopic image 4 from which it is estimated that the viewpoint of the endoscope 2 can be tracked.

On the other hand, in a case where the score received from the estimation unit 3B is equal to or greater than the reference score, the controller 3F performs control to resume the tracking of the viewpoint of the endoscope 2 since the viewpoint of the endoscope 2 can be tracked from the endoscopic image 4 acquired by the acquisition unit 3A.

Specifically, the estimation unit 3B estimates a viewpoint difference between the endoscopic image 4 and a virtual endoscopic image 6 which will be described below (simply, may be referred to as a "viewpoint difference between images"), in addition to the estimation of the trackability of the endoscope 2. The controller 3F tracks the viewpoint of the endoscope 2 using the viewpoint difference between the images estimated by the estimation unit 3B. Therefore, the controller 3F controls the estimation unit 3B such that the estimation unit 3B does not estimate the viewpoint difference between the images in a case where the received score is less than the reference score and controls the estimation unit 3B such that the estimation unit 3B estimates the viewpoint difference between the images in a case where the received score is equal to or greater than the reference score, thereby temporarily stopping and resuming the tracking of the viewpoint of the endoscope 2. In addition, a method for estimating the viewpoint difference between the images in the estimation unit 3B will be described in detail below. The estimation unit 3B notifies the controller 3F of the estimated viewpoint difference between the images.

Meanwhile, the bronchial model 5 representing the shape of the bronchus of the subject is used to notify the medical worker of the traveling direction of the endoscope 2. The bronchial model 5 is an example of a three-dimensional model that is generated in advance by, for example, computed tomography (CT) before endoscopy. Fig. 5 shows an example of the bronchial model 5. The bronchial model 5 is generated in advance for each subject and is stored in advance in the storage unit 3D.

The model acquisition unit 3C shown in Fig. 1 acquires the bronchial model 5 of the subject to be examined from the storage unit 3D.

The generation unit 3E generates an image in a case where the bronchial model 5 is viewed from a predetermined position inside the bronchial model 5, that is, the virtual endoscopic image 6, using the bronchial model 5 acquired by the model acquisition unit 3C. For example, a position where the distal end of the endoscope 2 is estimated to be present is used as the predetermined position inside the bronchial model 5. Fig. 6 is a diagram showing an example of the virtual endoscopic image 6 generated by the generation unit 3E using the bronchial model 5.

Since the bronchial model 5 is a three-dimensional model generated from data representing the shape of the bronchus of the subject, bubbles or foreign substances are not attached to the bronchial model 5 unlike the actual bronchus of the subject. In addition, since the virtual endoscopic image 6 is generated by data processing using the bronchial model 5, the virtual endoscopic image 6 is not out of focus, illumination light is not reflected, and whiteout or blackout does not occur. That is, the virtual endoscopic image 6 is an image that faithfully reproduces how the shape of the bronchial model 5 looks in a case where the bronchial model 5 is viewed from the designated viewpoint. Since the viewpoint of the endoscope 2 can be freely set in the bronchial model 5, the generation unit 3E can generate the virtual endoscopic image 6 from any viewpoint.

The estimation unit 3B estimates a viewpoint difference between the viewpoint at which the endoscopic image 4 has been obtained and the viewpoint at which the virtual endoscopic image 6 has been obtained from the deviation between the shapes of each bronchus or the textures of each bronchus included in the endoscopic image 4, from which the viewpoint of the endoscope 2 is estimated to be trackable, and any one of the virtual endoscopic images 6 generated by the generation unit 3E.

The controller 3F that has received the viewpoint difference between the images estimated by the estimation unit 3B specifies the viewpoint of the endoscope 2 based on the received viewpoint difference and determines a traveling direction in which the endoscope 2 reaches the target location using the bronchial model 5. In addition, the controller 3F controls the notification unit 3G such that the notification unit 3G notifies the medical worker of the determined traveling direction of the endoscope 2.

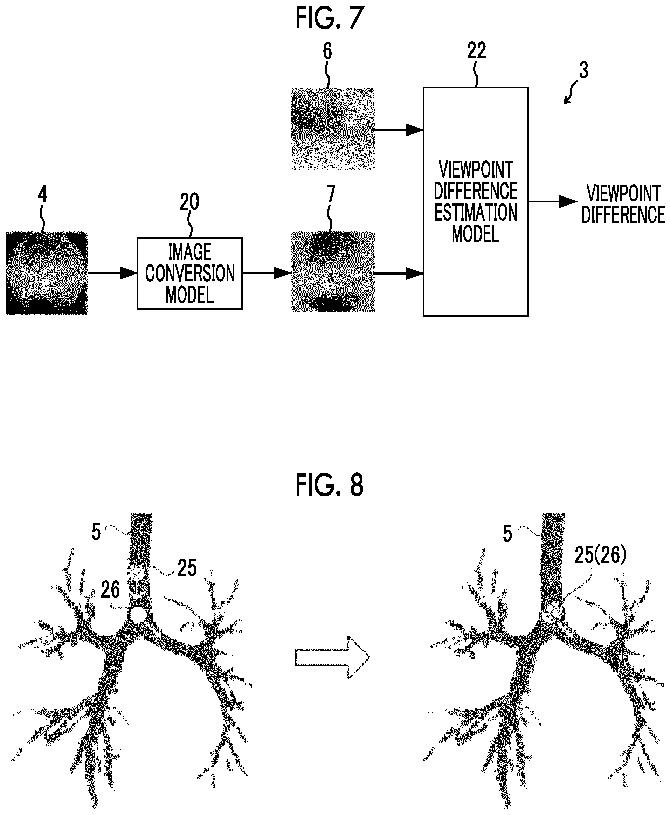

Next, a method for estimating the viewpoint difference between the images in the estimation unit 3B will be described. Fig. 7 is a diagram showing an example of the method for estimating the viewpoint difference between the images. The estimation unit 3B estimates the viewpoint difference between the images using a viewpoint difference estimation model 22 that has been subjected to machine learning in advance to output the viewpoint difference between the endoscopic image 4 and the virtual endoscopic image 6 for the endoscopic image 4 and the virtual endoscopic image 6 in a case where the bronchial model 5 is viewed from the designated viewpoint. In addition, instead of the endoscopic image 4, an image obtained from the endoscopic image 4 may be input to the viewpoint difference estimation model 22. The image obtained from the endoscopic image 4 is also an example of the endoscopic image 4.

As can be seen from the fact that the viewpoint difference estimation model 22 outputs the viewpoint difference between the input endoscopic image 4 and the input virtual endoscopic image 6, the virtual endoscopic image 6 input to the viewpoint difference estimation model 22 may be the virtual endoscopic image 6 obtained from a viewpoint different from the viewpoint at which the endoscopic image 4 has been captured.

The estimation unit 3B inputs the endoscopic image 4 to an image conversion model 20 to convert the endoscopic image 4 into a pseudo virtual endoscopic image 7.

The pseudo virtual endoscopic image 7 is an image obtained by converting the endoscopic image 4 into an expression format that looks like the virtual endoscopic image 6. Specifically, the pseudo virtual endoscopic image 7 is an image obtained by removing the texture of the bronchus or the unknown structure region from the endoscopic image 4 such that the shape of the bronchus is easier to ascertain than that in the endoscopic image 4.

The image conversion model 20 is a model generated in advance using, for example, a generative adversarial network (GAN) trained to output the pseudo virtual endoscopic image 7 similar to the virtual endoscopic image 6 from the endoscopic image 4 and is stored in the storage unit 3D in advance.

The estimation unit 3B inputs the virtual endoscopic image 6 and the pseudo virtual endoscopic image 7 obtained as described above to the viewpoint difference estimation model 22 to estimate the viewpoint difference between the endoscopic image 4, which is a generation source of the pseudo virtual endoscopic image 7, and the virtual endoscopic image 6. That is, the pseudo virtual endoscopic image 7 is also an example of the endoscopic image 4 input to the viewpoint difference estimation model 22.

Since the virtual endoscopic image 6 is an image generated from the bronchial model 5, the viewpoint of the virtual endoscopic image 6 is known to the controller 3F. Further, since the viewpoint difference between the endoscopic image 4 and the virtual endoscopic image 6 is notified from the estimation unit 3B, the controller 3F can specify the viewpoint of the endoscope 2 from the viewpoint of the virtual endoscopic image 6 and the viewpoint difference between the endoscopic image 4 and the virtual endoscopic image 6.

Fig. 8 is a diagram showing an example of the tracking of the viewpoint of the endoscope 2. In Fig. 8, a mark 25 indicates a predetermined position inside the bronchial model 5 (for example, a schematic position where the endoscope 2 is considered to be present), and a mark 26 indicates an actual position of the endoscope 2. The controller 3F can move the position of the mark 25 to the position of the mark 26 from the viewpoint difference between the images and take the same posture as the endoscope 2 whose position is indicated by the mark 26 on the bronchial model 5.

In a case where the viewpoint of the endoscope 2 indicated by the endoscopic image 4 is known, the virtual endoscopic image 6 obtained from the same viewpoint as the endoscope 2 is generated from the bronchial model 5, and it is possible to notify the medical worker of the generated virtual endoscopic image 6 together with the endoscopic image 4. That is, the information processing apparatus 3 can display the virtual endoscopic image 6 corresponding to the viewpoint of the endoscope 2 with the movement of the endoscope 2.

In addition, in a case where the viewpoint of the endoscope 2 indicated by the endoscopic image 4 is known, the information processing apparatus 3 can determine the traveling direction of the endoscope 2 for reaching the target location using the bronchial model 5.

In addition, the reason for using the image obtained by processing the endoscopic image 4 to be close to the virtual endoscopic image 6, such as the pseudo virtual endoscopic image 7, as the input of the viewpoint difference estimation model 22 instead of the endoscopic image 4 is to consider ease of the preparation of learning data (hereinafter, referred to as "viewpoint difference learning data") used for machine learning of the viewpoint difference estimation model 22.

For example, two endoscopic images 4 whose viewpoints deviate from each other and information related to the viewpoint difference between the endoscopic images 4 are used to perform the machine learning of the viewpoint difference estimation model 22. However, the endoscope 2 is being miniaturized such that the endoscope 2 can be inserted into as many bronchi as possible due to the characteristics of the endoscope 2. For example, it is difficult to separately attach sensors for measuring the viewpoint of the endoscope 2, such as a gyro sensor and a motion sensor. In addition, since the endoscope 2 is inserted into the body, it is possible to ascertain the viewpoint of the endoscope 2 only through the endoscopic image 4 with a limited angle of view, and it is also difficult to acquire the viewpoint difference from the endoscopic image 4.

In contrast, as described above, the viewpoint of the endoscope 2 can be freely set in the bronchial model 5. Since the bronchial model 5 is a three-dimensional model based on examination data of CT or the like, a correct viewpoint difference can be acquired by numerical calculation.

Therefore, in a case where a combination of two virtual endoscopic images 6 whose viewpoints deviate from each other and information related to the viewpoint difference between the virtual endoscopic images 6 is used as the viewpoint difference learning data, it is possible to more easily prepare a larger amount of viewpoint difference training data, as compared to a case where a combination of two endoscopic images 4 whose viewpoints deviate from each other and information related to the viewpoint difference between the endoscopic images 4 is used as the viewpoint difference learning data.

For the above reason, two virtual endoscopic images 6 having different viewpoints are used as the input data in the viewpoint difference learning data of the viewpoint difference estimation model 22. Therefore, in a case where the endoscopic image 4 is input to the viewpoint difference estimation model 22 without any change, the accuracy of estimation of the viewpoint difference by the viewpoint difference estimation model 22 may be reduced. Therefore, the process of converting the endoscopic image 4 into the pseudo virtual endoscopic image 7 and then inputting the pseudo virtual endoscopic image 7 to the viewpoint difference estimation model 22 is performed. Of course, the endoscopic image 4 may be input to the viewpoint difference estimation model 22 without any change. In this case, the machine learning of the viewpoint difference estimation model 22 may be performed using viewpoint difference learning data obtained by combining the endoscopic image 4, the virtual endoscopic image 6 having a viewpoint that deviates from the viewpoint of the endoscopic image 4, and information related to the viewpoint difference between the images.

The viewpoint difference estimation model 22 is a model generated in advance by the machine learning and is stored in the storage unit 3D in advance.

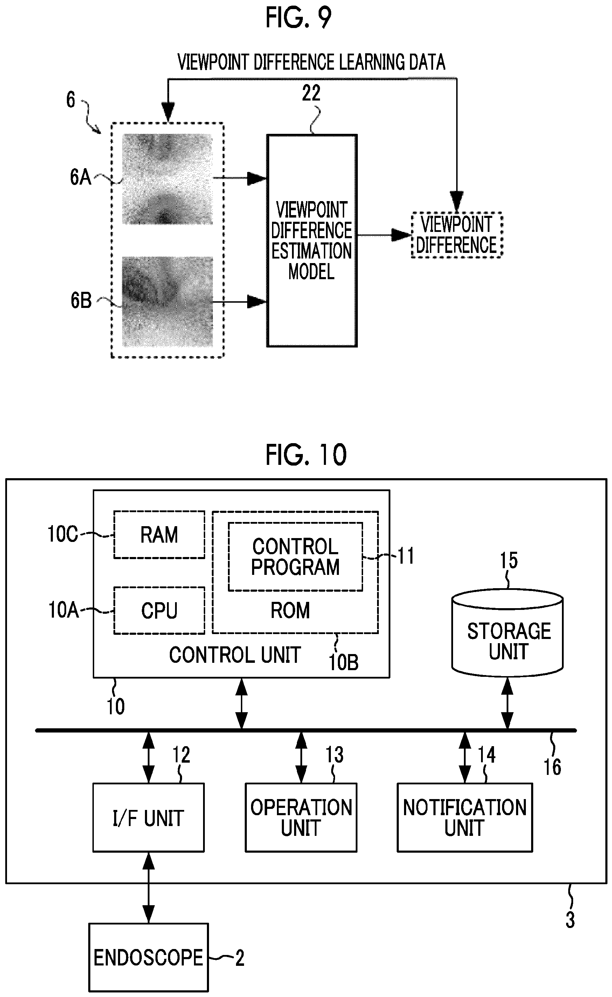

Fig. 9 is a diagram showing an example of the machine learning of the viewpoint difference estimation model 22.

A plurality of viewpoint difference learning data items in which the viewpoint difference between two virtual endoscopic images 6 having different viewpoints is associated with the virtual endoscopic images 6 are prepared in advance. In Fig. 9, a virtual endoscopic image 6A and a virtual endoscopic image 6B indicate the two virtual endoscopic images 6 having different viewpoints.

In addition, the machine learning of the viewpoint difference estimation model 22 is performed such that, in a case where the virtual endoscopic images 6A and 6B included in the viewpoint difference learning data are input, the viewpoint difference estimation model 22 outputs the viewpoint difference associated with each input. For example, a CNN or the like is used as the viewpoint difference estimation model 22. For example, deep learning is used as a machine learning method.

That is, in the viewpoint difference learning data, the virtual endoscopic images 6A and 6B are input data, and the viewpoint difference is training data.

In addition, the machine learning of the viewpoint difference estimation model 22 may be performed using a range of the bronchial model 5 (referred to as a "partial bronchial model") viewed from each viewpoint, instead of any one of the virtual endoscopic image 6A or the virtual endoscopic image 6B. That is, any one of the virtual endoscopic images 6 may be represented as three-dimensional data. In this case, in the method for estimating the viewpoint difference between the images shown in Fig. 7, the partial bronchial model at a designated viewpoint is used instead of the virtual endoscopic image 6.

The machine learning of various models, such as the image conversion model 20, the trackability estimation model 21, and the viewpoint difference estimation model 22, and the generation of the bronchial model 5 are not necessarily performed by the information processing apparatus 3 and may be performed by another computer having a higher processing capacity than the information processing apparatus 3. In this case, the information processing apparatus 3 acquires various models generated by another computer from another computer and uses the various models.

The information processing apparatus 3 shown in Fig. 1 that has these functions is configured by, for example, a computer. Fig. 10 is a diagram showing an example of a configuration of the information processing apparatus 3 configured by the computer.

As shown in Fig. 10, the information processing apparatus 3 comprises a control unit 10, an interface (I/F) unit 12, an operation unit 13, a notification unit 14, and a storage unit 15. The control unit 10, the I/F unit 12, the operation unit 13, the notification unit 14, and the storage unit 15 are connected to each other via a bus 16 such that they can transmit and receive various types of information.

The control unit 10 controls the operation of the information processing apparatus 3 based on an instruction from the medical worker. The control unit 10 is an example of a processor and comprises a central processing unit (CPU) 10A that is in charge of processes of each functional unit of the information processing apparatus 3 shown in Fig. 1, a read only memory (ROM) 10B, and a random access memory (RAM) 10C. The ROM 10B stores in advance various programs including a control program 11 that is read by a CPU 10A to track the viewpoint of the endoscope 2 and various parameters that are referred to by the CPU 10A to control the operation of the information processing apparatus 3. The RAM 10C is used as a temporary work area of the CPU 10A.

The I/F unit 12 transmits and receives various types of information to and from the endoscope 2 using wireless communication or wired communication. The information processing apparatus 3 acquires the endoscopic image 4 from the endoscope 2 via the I/F unit 12.

The operation unit 13 is used, for example, by the medical worker to input an instruction related to the tracking of the viewpoint of the endoscope 2, various types of information, and the like. There is no restriction on the operation form of the operation unit 13. For example, it is possible to receive the operations by a switch, a touch panel, a touch pen, a keyboard, a mouse, and the like.

The notification unit 14 notifies the medical worker of the information processed by the control unit 10, such as the endoscopic image 4, the virtual endoscopic image 6, and the information related to the tracking of the viewpoint of the endoscope 2.

The notification of the information is to enable the medical worker to recognize the information. Therefore, for example, all of a form in which information is displayed on a display (not shown) which is an example of the display device, a form in which information is printed on a recording medium, such as paper, by an image forming apparatus (not shown), and a form in which information is notified by voice through a speaker (not shown) are examples of the notification of the information by the notification unit 14. In addition, a form in which information is transmitted through the I/F unit 12 is also an example of the notification of the information. The information processing apparatus 3 according to the embodiment of the present disclosure notifies of the information by the display of the information on the display and by voice notification.

The storage unit 15 stores, for example, the bronchial model 5 and various models, such as the image conversion model 20, the trackability estimation model 21, and the viewpoint difference estimation model 22. The storage unit 15 is an example of a storage device that retains stored information even in a case where power supplied to the storage unit 15 is cut off. For example, a semiconductor memory, such as a solid state drive (SSD), is used as the storage unit 15. However, a hard disk may be used.

Next, a process of the information processing apparatus 3 that tracks the viewpoint of the endoscope 2 will be described in detail.

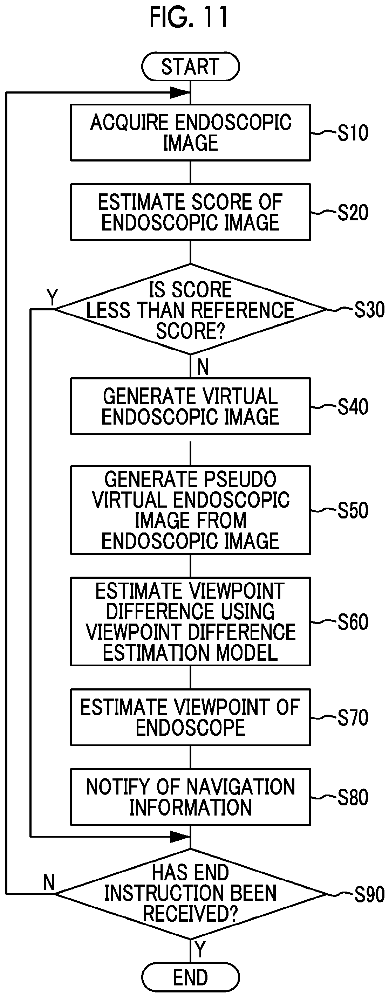

Fig. 11 is a flowchart showing an example of a flow of a tracking process executed by the information processing apparatus 3 in a case where an instruction to track the viewpoint of the endoscope 2 is received by the operation of the operation unit 13 by the medical worker. The CPU 10A of the information processing apparatus 3 reads the control program 11 from the ROM 10B and executes the tracking process. The control program 11 is an example of an information processing program according to the embodiment of the present disclosure.

In addition, it is assumed that the medical worker sets an initial viewpoint of the endoscope 2 in the bronchial model 5 in advance. The initial viewpoint of the endoscope 2 set by the medical worker may be a viewpoint different from the actual viewpoint of the endoscope 2. Alternatively, the CPU 10A may randomly set the initial viewpoint in the bronchial model 5.

In addition, it is assumed that the storage unit 15 stores the bronchial model 5 of the subject, the image conversion model 20, the trackability estimation model 21, the viewpoint difference estimation model 22, and the reference score in advance.

First, in Step S10, the CPU 10A acquires the endoscopic image 4 from the endoscope 2 via the I/F unit 12. The endoscopic image 4 may be a color image or a monochrome image.

In Step S20, the CPU 10A inputs the endoscopic image 4 acquired by the process in Step S10 to the trackability estimation model 21 to estimate the score of the endoscopic image 4.

In Step S30, the CPU 10A determines whether or not the score of the endoscopic image 4 estimated by the process in Step S20 is less than the reference score.

In a case where the score of the endoscopic image 4 is equal to or greater than the reference score, the process proceeds to Step S40. In this case, since the viewpoint of the endoscope 2 can be tracked, in Step S40, the CPU 10A generates the virtual endoscopic image 6 in a case where the bronchial model 5 is viewed from the set viewpoint.

In Step S50, the CPU 10A inputs the endoscopic image 4 acquired by the process in Step S10 to the image conversion model 20 to generate the pseudo virtual endoscopic image 7 corresponding to the endoscopic image 4.

In Step S60, the CPU 10Ainputs the virtual endoscopic image 6 generated by the process in Step S40 and the pseudo virtual endoscopic image 7 generated by the process in Step S50 to the viewpoint difference estimation model 22 to estimate the viewpoint difference between the endoscopic image 4 acquired by the process in Step S10 and the virtual endoscopic image 6 generated by the process in Step S40.

In Step S70, the CPU 10A reflects the viewpoint difference estimated by the process in Step S60 in the viewpoint set in the bronchial model 5 to estimate the viewpoint of the endoscope 2.

In Step S80, the CPU 10A sets the viewpoint of the endoscope 2 specified by the process in Step S70 in the bronchial model 5 to update the viewpoint of the endoscope 2 in the bronchial model 5. The CPU 10A searches for a route from the updated viewpoint of the endoscope 2 to the target location using the bronchial model 5. For example, a known search method, such as a binary search method, can be used to search for the route to the target location.

The CPU 10A notifies the medical worker of the traveling direction of the endoscope 2, such as a "right direction" or a "downward direction", by voice through the speaker based on the search result of the route. In this way, the CPU 10A notifies the medical worker of the navigation information for the endoscope 2 to reach the target location.

In addition, the CPU 10A may generate the virtual endoscopic image 6 in a case where the bronchial model 5 is viewed from the updated viewpoint of the endoscope 2 in the bronchial model 5 and may display the generated virtual endoscopic image 6 side by side with, for example, the endoscopic image 4 acquired by the process in Step S10 on the display. Therefore, the virtual endoscopic image 6 corresponding to the viewpoint of the endoscope 2 is displayed according to a change in the viewpoint of the endoscope 2.

After the process in Step S80 is ended or in a case where it is determined in the determination process in Step S30 that the score of the endoscopic image 4 is less than the reference score, the CPU 10A proceeds to Step S90. That is, in a case where the score of the endoscopic image 4 is less than the reference score, the CPU 10A does not track the viewpoint of the endoscope 2.

In Step S90, the CPU 10A determines whether or not an instruction to end the tracking process has been received from the user. In a case where the end instruction has not been received, the CPU 10A proceeds to Step S10 and acquires a new endoscopic image 4 from the endoscope 2. That is, the information processing apparatus 3 tracks the viewpoint of the endoscope 2 with the movement of the endoscope 2 and notifies the medical worker of the navigation information of the endoscope 2 until the end instruction is received from the user.

On the other hand, in a case where the end instruction has been received from the user, the tracking processing shown in Fig. 11 is ended.

As described above, according to the information processing apparatus 3 of the first embodiment, in a case where it is determined that the viewpoint of the endoscope 2 is not trackable from the endoscopic image 4, the tracking of the viewpoint of the endoscope 2 is temporarily stopped. Even during the period for which the tracking of the viewpoint of the endoscope 2 is temporarily stopped, the information processing apparatus 3 continues to acquire the endoscopic image 4 and estimates the trackability of the endoscope 2 for each of the acquired endoscopic images 4 until the end instruction is received from the user. In a case where the endoscopic image 4 from which the viewpoint of the endoscope 2 can be tracked is acquired, the information processing apparatus 3 resumes the tracking of the viewpoint of the endoscope 2.

Therefore, the information processing apparatus 3 can prevent the notification of the navigation information based on the erroneous estimation of the viewpoint, as compared with a case where the viewpoint of the endoscope 2 is estimated from all of the endoscopic images 4 without determining the trackability of the endoscope 2.

In addition, in the tracking processing shown in Fig. 11, before the CPU 10A tracks the viewpoint of the endoscope 2 using the process in Steps S40 to S70, it is estimated whether or not the viewpoint of the endoscope 2 can be tracked by the determination process in Step S30.

However, the information processing apparatus 3 may first track the viewpoint of the endoscope 2 and then estimate whether or not the viewpoint of the endoscope 2 is correctly tracked.

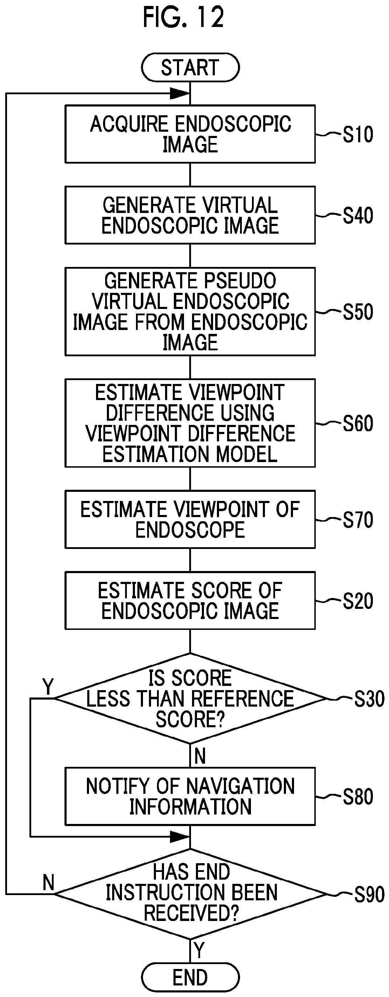

Fig. 12 is a flowchart showing a modification example of the flow of the tracking process executed by the information processing apparatus 3 in a case where an instruction to start the tracking of the viewpoint of the endoscope 2 has been received by the operation of the medical worker through the operation unit 13.

The flowchart shown in Fig. 12 differs from the flowchart of the tracking process shown in Fig. 11 in that the process in Steps S40 to S70 is executed between Step S10 and Step S20. That is, the CPU 10A first tracks the viewpoint of the endoscope 2 using the endoscopic image 4 acquired from the endoscope 2 and then estimates the trackability of the endoscope 2. In a case where the score of the endoscopic image 4 used for tracking the viewpoint is less than the reference score, the CPU 10A determines that an error of the tracked viewpoint is not included in the allowable range and does not notify the navigation information of the endoscope 2. On the other hand, in a case where the score of the endoscopic image 4 used for tracking the viewpoint is equal to or greater than the reference score, the CPU 10A determines that the error of the tracked viewpoint is within the allowable range and notifies the navigation information of the endoscope 2.

As described above, there is no restriction on the estimation time of the trackability of the endoscope 2 in the tracking process. The tracking process may be performed in any period of the tracking process after the endoscopic image 4 is acquired. Therefore, for example, in a case where the estimation of the trackability of the endoscope 2 in Step S20 is performed in parallel with the process in Steps S40 to S70 shown in Fig. 12, it is possible to shorten the time from the acquisition of the endoscopic image 4 to the notification of the navigation information, as compared to a case where the processes are sequentially executed.

In addition, the image used by a CPU 10A to estimate the trackability of the endoscope 2 is not limited to the endoscopic image 4. The CPU 10A may estimate the trackability of the endoscope 2 using the image generated from the endoscopic image 4 or another image other than the endoscopic image 4 in which the shape of the bronchus can be recognized. In addition, the CPU 10A may combine a plurality of types of images to estimate the trackability of the endoscope 2.

For example, the CPU 10A may estimate the trackability of the endoscope 2 using a depth image corresponding to the endoscopic image 4. The depth image corresponding to the endoscopic image 4 is, for example, an image in which the shape of the bronchus at a position where the distal end of the endoscope 2 is present is represented as distance data from an entrance of the bronchus using distance data obtained from a depth sensor that measures the distance from the entrance of the bronchus. For example, a stereo camera, a time-of-flight (TOF) sensor, a light detection and ranging (LiDAR) sensor, or a photoelectric sensor is used as the depth sensor. In addition, the CPU 10A may combine the endoscopic image 4 with the depth image to estimate the trackability of the endoscope 2 or may estimate the trackability of the endoscope 2 using positional information of the unknown structure region or brightness information of the endoscopic image 4.

In this case, machine learning may be performed on the trackability estimation model 21, using the estimation learning data in which the image used for estimating the trackability of the endoscope 2 is used as input data and the score is used as training data.

In addition, the example in which the trackability of the endoscope 2 is estimated using the score has been described above. However, the trackability of the endoscope 2 may be estimated using attributes other than the score.

For example, the information processing apparatus 3 may represent the trackability of the endoscope 2 using a plurality of classes, such as "trackable", "untrackable", and "unestimateable" classes, and may classify the endoscopic image 4 into any class using the trackability estimation model 21. In a case where the endoscopic image 4 is classified into the trackable class, the information processing apparatus 3 may estimate that the viewpoint of the endoscope 2 is trackable. In a case where the endoscopic image 4 is classified into the untrackable class or the unestimateable class, the information processing apparatus 3 may estimate that the viewpoint of the endoscope 2 is untrackable.

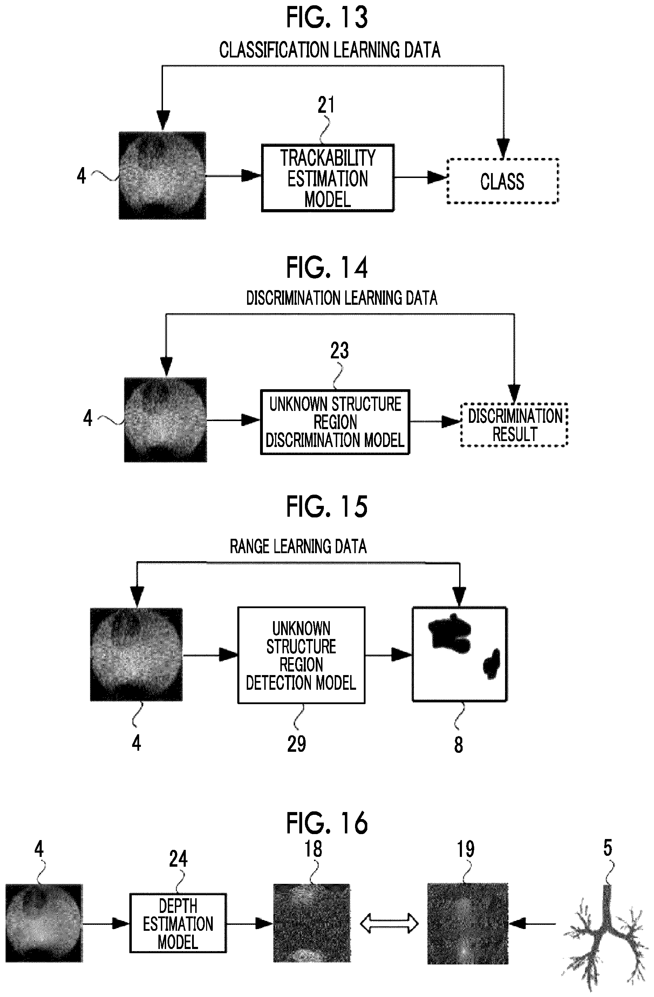

Fig. 13 is a diagram showing an example of the machine learning of the trackability estimation model 21 that classifies the endoscopic image 4. As shown in Fig. 13, a plurality of classification learning data items in which any one of the "trackable", "untrackable", or "unestimateble" classes is associated with the endoscopic image 4 are prepared in advance. In a case where the endoscopic image 4 included in the classification learning data is input, the machine learning of the trackability estimation model 21 is performed such that the endoscopic image 4 is classified into the class associated with the endoscopic image 4. That is, in the classification learning data of the trackability estimation model 21, the endoscopic image 4 is input data, and the class into which the endoscopic image 4 is classified is training data. The medical worker associates the endoscopic image 4 with the class based on, for example, the state of the unknown structure region, such as the presence or absence, type, and range of the unknown structure region in the endoscopic image 4.

In addition, the information processing apparatus 3 may estimate the trackability of the endoscope 2 using an unknown structure region discrimination model 23 instead of the trackability estimation model 21.

For example, the information processing apparatus 3 estimates the trackability of the endoscope 2 based on the type of the unknown structure region that is included in the endoscopic image 4 and that affects the tracking of the viewpoint of the endoscope 2.

The unknown structure region discrimination model 23 is a model that discriminates the type of the unknown structure region included in the endoscopic image 4. The unknown structure region discrimination model 23 outputs the presence or absence of unknown structure region, such as the presence or absence of bubbles or foreign substances, the presence or absence of defocus, and the presence or absence of whiteout or blackout, from the endoscopic image 4 and outputs the size of each unknown structure region. The information processing apparatus 3 may estimate the trackability of the endoscope 2 with reference to a determination table (not shown), in which the trackability of the endoscope 2 has been defined in advance, for each combination of the type and size of the unknown structure region included in the endoscopic image 4 discriminated by the unknown structure region discrimination model 23. For example, the determination table is stored in the storage unit 15 in advance.

Fig. 14 is a diagram showing an example of machine learning of the unknown structure region discrimination model 23 that discriminates the type and size of the unknown structure region from the endoscopic image 4. A plurality of discrimination learning data items in which the type and size of the unknown structure region included in the endoscopic image 4 are associated with the endoscopic image 4 captured in advance are prepared in advance. In addition, the machine learning of the unknown structure region discrimination model 23 is performed such that, in a case where the endoscopic image 4 included in the discrimination learning data is input, the unknown structure region discrimination model 23 outputs the type and size of the unknown structure region associated with the input endoscopic image 4. That is, in the discrimination learning data, the endoscopic image 4 is input data, and the type and size of the unknown structure region are training data. For example, a CNN or the like is used as the unknown structure region discrimination model 23. For example, deep learning is used as a machine learning method.

For example, the medical worker views the endoscopic image 4 and associates the type and size of the unknown structure region to generate the type and size of the unknown structure region in the discrimination learning data. The medical worker may associate the position of the unknown structure region as the size of the unknown structure region.

In addition, the information processing apparatus 3 may estimate the trackability of the endoscope 2 using an unknown structure region detection model 29 instead of the trackability estimation model 21.

For example, the information processing apparatus 3 estimates the trackability of the endoscope 2 based on the range of the unknown structure region included in the endoscopic image 4.

The unknown structure region detection model 29 is a model that detects the range of the unknown structure region included in the endoscopic image 4. The range of the unknown structure region in the endoscopic image 4 is indicated by, for example, the positional information of the unknown structure region. The information processing apparatus 3 calculates the area of the unknown structure region using the range of the unknown structure region included in the endoscopic image 4 detected by the unknown structure region detection model 29 and estimates that the viewpoint of the endoscope 2 can be tracked in a case where the area of the unknown structure region is less than a threshold value. The area of the unknown structure region may be represented by, for example, the number of pixels constituting the unknown structure region in the endoscopic image 4.

The unknown structure region detection model 29 is, for example, a model generated in advance by machine learning and is stored in the storage unit 3D in advance.

Fig. 15 is a diagram showing an example of the machine learning of the unknown structure region detection model 29. A plurality of learning data items (referred to as "range learning data"), in which mask information 8 in which the range of the unknown structure region of the endoscopic image 4 is painted is associated with the endoscopic image 4 captured in advance, are prepared in advance. In addition, the machine learning of the unknown structure region detection model 29 is performed such that, in a case where the endoscopic image 4 included in the range learning data is input, the unknown structure region detection model 29 outputs the same range as the range of the mask information 8 associated with the input endoscopic image 4. That is, in the range learning data, the endoscopic image 4 is input data, and the mask information 8 is training data. For example, a creator of the range learning data views the endoscopic image 4 and paints a range that is considered to be the unknown structure region to generate the mask information 8 in the range learning data.

In addition, the information processing apparatus 3 may calculate the area of the entire endoscopic image 4 and estimate the trackability of the endoscope 2 with reference to a determination table in which the trackability of each endoscope 2 has been defined in advance according to the proportion of the area of the unknown structure region to the area of the entire endoscopic image 4. The area of the entire endoscopic image 4 may be represented by, for example, the number of pixels constituting the endoscopic image 4.

As described above, the trackability of the endoscope 2 can also be estimated using attributes other than the score.

### Second Embodiment

In the first embodiment, the example has been described in which the viewpoint difference is estimated using the viewpoint difference estimation model 22 generated by machine learning and the viewpoint of the endoscope 2 is tracked based on the estimated viewpoint difference.

However, a method for tracking the viewpoint of the endoscope 2 is not limited to the method using the viewpoint difference estimation model 22 generated by machine learning. Any method may be used as long as it can track the viewpoint of the endoscope 2.

In the second embodiment, the information processing apparatus 3 that estimates a viewpoint between images based on the similarity of a depth image corresponding to the endoscopic image 4 and tracks the viewpoint of the endoscope 2 based on the estimated viewpoint will be described.

An example of a functional configuration of the information processing apparatus 3 according to the second embodiment is the same as the example of the functional configuration of the information processing apparatus 3 shown in Fig. 1, and the information processing apparatus 3 is configured using the computer shown in Fig. 10.

The information processing apparatus 3 estimates the viewpoint of the endoscope 2 from a similarity between a depth image (hereinafter, referred to as an "endoscopic depth image 18") corresponding to the endoscopic image 4 and a depth image generated using the bronchial model 5. The similarity is represented by, for example, a numerical value. For example, it is assumed that, as the numerical value of the similarity is larger, the similarity between the images is higher.

Therefore, the information processing apparatus 3 generates the endoscopic depth image 18 from the endoscopic image 4, which will be described below. In addition, the information processing apparatus 3 generates a depth image (hereinafter, referred to as a "virtual depth image 19") at each position inside the bronchial model 5.

The information processing apparatus 3 calculates the similarity between each generated virtual depth image 19 and the endoscopic depth image 18 and specifies a virtual depth image 19 most similar to the endoscopic depth image 18 from the virtual depth images 19. In addition, the information processing apparatus 3 searches for a viewpoint at which an image having the highest similarity to the endoscopic image 4, which is the generation source of the endoscopic depth image 18, is obtained at the position indicated by the specified virtual depth image 19 and estimates the viewpoint of the endoscope 2. That is, the viewpoint at which the image having the highest similarity to the endoscopic image 4 is obtained at the position indicated by the virtual depth image 19 most similar to the endoscopic depth image 18 is the viewpoint of the endoscope 2.

In addition, for example, a known image recognition method that compares the feature amounts of images to calculate the similarity can be applied to calculate the similarity between the endoscopic depth image 18 and the virtual depth image 19 (simply referred to as a "similarity between images"). Further, a similarity estimation model which is a model generated by machine learning and outputs the similarity between the images may be used to calculate the similarity between the images.

Fig. 16 is a diagram showing an example of a method for estimating the similarity between the images in the information processing apparatus 3 according to the second embodiment. It is assumed that the generation unit 3E generates the virtual depth image 19 at each position of the bronchial model 5 before the similarity is estimated.

For example, the estimation unit 3B inputs the endoscopic image 4 to a depth estimation model 24 to convert the endoscopic image 4 into the endoscopic depth image 18. The depth estimation model 24 is, for example, a model generated in advance using a GAN that has been trained to output the endoscopic depth image 18 from the endoscopic image 4 and is stored in advance in the storage unit 3D.

The estimation unit 3B calculates the similarity between each virtual depth image 19 generated by the generation unit 3E and the endoscopic depth image 18 to estimate the viewpoint of the endoscope 2.

Next, a process of the information processing apparatus 3 that tracks the viewpoint of the endoscope 2 based on the similarity between the images will be described in detail.

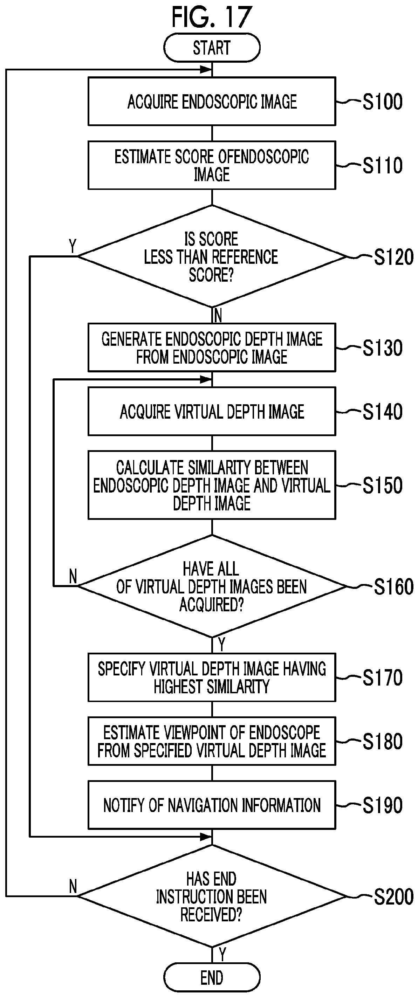

Fig. 17 is a flowchart showing an example of a flow of a tracking process executed by the information processing apparatus 3 in a case where an instruction to track the viewpoint of the endoscope 2 is received by the operation of the operation unit 13 by the medical worker. The CPU 10A of the information processing apparatus 3 reads the control program 11 from the ROM 10B and executes the tracking process. It is assumed that the storage unit 15 stores the bronchial model 5 of the subject, the virtual depth image 19 at each position inside the bronchial model 5 of the subject, the trackability estimation model 21, the depth estimation model 24, and the reference score in advance.

First, in Step S100, the CPU 10A acquires the endoscopic image 4 from the endoscope 2 via the I/F unit 12.

In Step S110, the CPU 10A inputs the endoscopic image 4 acquired by the process in Step S100 to the trackability estimation model 21 to estimate the score of the endoscopic image 4.

In Step S120, the CPU 10A determines whether or not the score of the endoscopic image 4 estimated by the process in Step S110 is less than the reference score.

In a case where the score of the endoscopic image 4 is equal to or greater than the reference score, the CPU 10A proceeds to Step S130. In this case, since the viewpoint of the endoscope 2 can be tracked, in Step S130, the CPU 10A inputs the endoscopic image 4 acquired by the process in Step S100 to the depth estimation model 24 to generate the endoscopic depth image 18.

In Step S140, the CPU 10A acquires any one virtual depth image 19 from the plurality of virtual depth images 19 stored in the storage unit 15.

In Step S150, the CPU 10A calculates the similarity between the endoscopic depth image 18 generated by the process in Step S130 and the virtual depth image 19 acquired by the process in Step S140 and stores the calculated similarity in the RAM 10C in association with the virtual depth image 19 used to calculate the similarity.

In Step S160, the CPU 10A determines whether or not all of the virtual depth images 19 stored in the storage unit 15 have been acquired. In a case where all of the virtual depth images 19 have not been acquired, the CPU 10A proceeds to Step S140 and acquires one virtual depth image 19 that has not been acquired. That is, the CPU 10A repeats the processes in Steps S140 to S160 until it is determined in the determination process in Step S160 that all of the virtual depth images 19 have been acquired and associates the similarity between the endoscopic depth image 18 and each virtual depth image 19 with each virtual depth image 19.

On the other hand, in a case where it is determined in the determination process in Step S160 that all of the virtual depth images 19 have been acquired, the CPU 10A proceeds to Step S170.

In Step S170, the CPU 10A specifies a virtual depth image 19, with which the highest similarity is associated, among all of the virtual depth images 19 as the virtual depth image 19 that is most similar to the endoscopic depth image 18 with reference to the similarities associated with each virtual depth image 19 stored in the RAM 10C.

In Step S180, the CPU 10A searches for a viewpoint at which an image having the highest similarity to the endoscopic image 4, which is the generation source of the endoscopic depth image 18, is obtained at the position of the bronchial model 5 indicated by the virtual depth image 19 specified by the process in Step S170 to estimate the viewpoint of the endoscope 2.

In Step S190, the CPU 10A sets the viewpoint of the endoscope 2 specified by the process in Step S180 in the bronchial model 5 to update the viewpoint of the endoscope 2 in the bronchial model 5. The CPU 10A searches for a route from the updated viewpoint of the endoscope 2 to the target location using the bronchial model 5 and notifies the medical worker of the traveling direction of the endoscope 2, such as a "right direction" or a "downward direction", as the navigation information by voice based on the search result of the route.

After the process in Step S190 is ended, or in a case where it is determined in the determination process in Step S120 that the score of the endoscopic image 4 is less than the reference score, the CPU 10A proceeds to Step S200. That is, in a case where the score of the endoscopic image 4 is less than the reference score, the CPU 10A does not track the viewpoint of the endoscope 2.

In Step S200, the CPU 10A determines whether or not an instruction to end the tracking process has been received from the user. In a case where the end instruction has not been received, the CPU 10A proceeds to Step S100 and acquires a new endoscopic image 4 from the endoscope 2. That is, the information processing apparatus 3 tracks the viewpoint of the endoscope 2 with the movement of the endoscope 2 and notifies the medical worker of the navigation information of the endoscope 2 until the end instruction is received from the user.

On the other hand, in a case where the end instruction has been received from the user, the CPU 10A ends the tracking process shown in Fig. 17.

As described above, according to the information processing apparatus 3 of the second embodiment, the viewpoint of the endoscope 2 is estimated based on the similarity between the endoscopic depth image 18 and the virtual depth image 19. Then, the viewpoint of the endoscope 2 is tracked. Even in this configuration, in a case where the information processing apparatus 3 determines that it is not possible to track the viewpoint of the endoscope 2 from the endoscopic image 4, the information processing apparatus 3 temporarily stops the tracking of the viewpoint of the endoscope 2. As already described, it goes without saying that, in the tracking process shown in Fig. 17, the viewpoint of the endoscope 2 may be estimated based on the similarity between the images and then the trackability of the endoscope 2 may be estimated.

In addition, supplementary information related to the position of the endoscope 2 may be added as the input data of the viewpoint difference learning data of the viewpoint difference estimation model 22 in the information processing apparatus 3 according to the first embodiment. In this case, the information processing apparatus 3 estimates the viewpoint difference of the endoscope 2 using the endoscopic image 4, the virtual endoscopic image 6, the supplementary information, and the viewpoint difference estimation model 22.

For example, a depth image corresponding to the endoscopic image 4 is used as the supplementary information. In addition, an insertion length of the endoscope 2 into the bronchus at the time when the endoscopic image 4 is captured may be used as the supplementary information.

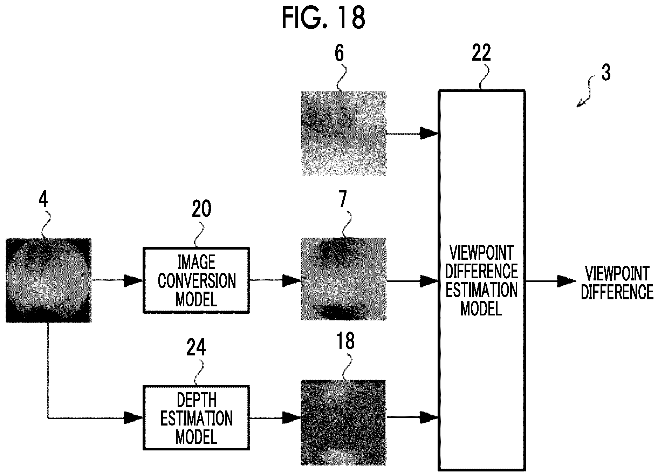

Fig. 18 is a diagram showing an example of a situation in which the supplementary information is added in the method for estimating the viewpoint difference of the endoscope 2 shown in Fig. 7. In the example shown in Fig. 18, the endoscopic depth image 18 generated from the endoscopic image 4 by the depth estimation model 24 is input as the supplementary information to the viewpoint difference estimation model 22.

In a case where the supplementary information is added to the input of the viewpoint difference estimation model 22, the accuracy of estimation of the viewpoint difference may be improved as compared to a case where the viewpoint difference of the endoscope 2 is estimated without using the supplementary information.

One form of the information processing system 1 has been described above using the embodiment. However, the disclosed form of the information processing system 1 is an example. The form of the information processing system 1 is not limited to the range described in the embodiment. Various modifications and improvements can be added to the embodiments without departing from the gist of the present disclosure, and the embodiments to which the modifications or improvements are added are also included in the technical scope of the present disclosure.

For example, the internal processing order in the flowcharts of each of the tracking processes shown in Figs. 11, 12, and 17 may be changed without departing from the gist of the present disclosure.

In each of the above-described embodiments, the form in which each tracking process is implemented by software processing has been described as an example. However, the same processes as those in the flowcharts of each tracking process may be performed by hardware. In this case, the processing speed can be increased as compared to a case where each tracking process is implemented by the software processing.

In the above-described embodiments, the term "processor" refers to hardware in a broad sense. Examples of the processor include general processors (for example, the CPU 10A) and dedicated processors (for example, a graphics processing unit: GPU, an application specific integrated circuit: ASIC, a field programmable gate array: FPGA, and a programmable logic device).

Further, the operation of the processor in each of the above-described embodiments may be performed not only by one processor but also by cooperation of a plurality of processors provided at physically separated positions. In addition, the order of the operations of the processor is not limited to only the order described in the embodiments and may be changed as appropriate.

In each of the above-described embodiments, the example in which the control program 11 is stored in the ROM 10B has been described. However, the storage destination of the control program 11 is not limited to the ROM 10B. The control program 11 can also be provided in a form in which the control program 11 is recorded in a computer-readable storage medium.

For example, the control program 11 may be provided in a form in which the control program 11 is recorded in an optical disk such as a compact disk read only memory (CD-ROM), a digital versatile disk read only memory (DVD-ROM), or a Blu-ray disc. In addition, the control program 11 may be provided in a form in which the control program 11 is recorded in a portable semiconductor memory such as a universal serial bus (USB) memory or a memory card. The ROM 10B, the CD-ROM, the DVD-ROM, the Blu-ray disc, the USB, and the memory card are examples of a non-transitory storage medium.

Further, the control unit 10 may download the control program 11 from an external apparatus connected to the communication line via the I/F unit 12 and store the downloaded control program 11 in the ROM 10B of the control unit 10.

For the above-described embodiments, the following supplementary notes are further disclosed.

### Supplementary Note 1

An information processing apparatus comprising:
an acquisition unit that acquires an endoscopic image of a living body;
an estimation unit that estimates whether or not a viewpoint of an endoscope that has captured the endoscopic image is trackable from the endoscopic image acquired by the acquisition unit; and
a controller that, in a case where the estimation unit estimates that the viewpoint of the endoscope is not trackable, performs control to temporarily stop tracking of the viewpoint of the endoscope until the acquisition unit acquires the endoscopic image from which the estimation unit estimates that the viewpoint of the endoscope is trackable.

### Supplementary Note 2

The information processing apparatus according to Supplementary Note 1,
wherein the estimation unit estimates whether or not the viewpoint of the endoscope that has captured the endoscopic image is trackable, based on a score indicating a degree of trackability of the viewpoint of the endoscope obtained from the endoscopic image.

### Supplementary Note 3

The information processing apparatus according to Supplementary Note 1,
wherein the estimation unit estimates whether or not the viewpoint of the endoscope that has captured the endoscopic image is trackable, based on a classification result of classifying the endoscopic image into any of a plurality of classes including a trackable class and an untrackable class.

### Supplementary Note 4

The information processing apparatus according to Supplementary Note 1,
wherein the estimation unit estimates whether or not the viewpoint of the endoscope that has captured the endoscopic image is trackable, using a type of an unknown structure region that is included in the endoscopic image and that is determined in advance to affect the tracking of the viewpoint of the endoscope.

### Supplementary Note 5

The information processing apparatus according to any one of Supplementary Notes 1 to 4,
wherein the estimation unit estimates whether or not the viewpoint of the endoscope is trackable before the controller tracks the viewpoint of the endoscope.

### Supplementary Note 6

The information processing apparatus according to any one of Supplementary Notes 1 to 4,
wherein the estimation unit estimates whether or not the viewpoint of the endoscope is trackable after the controller tracks the viewpoint of the endoscope.

### Supplementary Note 7

The information processing apparatus according to any one of Supplementary Notes 1 to 6,
wherein the controller controls the acquisition unit such that the acquisition unit continues to acquire the endoscopic image and controls the estimation unit such that the estimation unit continues to estimate whether or not the viewpoint of the endoscope is trackable from the endoscopic image acquired by the acquisition unit even during a period for which the tracking of the viewpoint of the endoscope is temporarily stopped.

### Supplementary Note 8

The information processing apparatus according to Supplementary Note 7,
wherein the controller performs control to resume the tracking of the viewpoint of the endoscope in a case where the estimation unit estimates that the viewpoint of the endoscope is trackable for a new endoscopic image acquired by the acquisition unit during the period for which the tracking of the viewpoint of the endoscope is temporarily stopped.

### Supplementary Note 9

An information processing program causing a computer to execute a process comprising:
acquiring an endoscopic image of a living body;
estimating whether or not a viewpoint of an endoscope that has captured the endoscopic image is trackable from the acquired endoscopic image; and
in a case where it is estimated that the viewpoint of the endoscope is not trackable, temporarily stopping tracking of the viewpoint of the endoscope until the endoscopic image, from which it is estimated that the viewpoint of the endoscope is trackable, is acquired.

## Claims

1. An information processing apparatus (3) comprising:
an acquisition unit (3A) that acquires an endoscopic image (4) of a living body;
an estimation unit (3B) that estimates whether or not a viewpoint of an endoscope (2) that has captured the endoscopic image (4) is trackable from the endoscopic image (4) acquired by the acquisition unit (3A); and
a controller (3F) that, in a case where the estimation unit (3B) estimates that the viewpoint of the endoscope (2) is not trackable, performs control to temporarily stop tracking of the viewpoint of the endoscope (2) until the acquisition unit (3A) acquires the endoscopic image (4) from which the estimation unit (3B) estimates that the viewpoint of the endoscope (2) is trackable.

2. The information processing apparatus (3) according to claim 1,
wherein the estimation unit (3B) estimates whether or not the viewpoint of the endoscope (2) that has captured the endoscopic image (4) is trackable, based on a score indicating a degree of trackability of the viewpoint of the endoscope (2) obtained from the endoscopic image (4).

3. The information processing apparatus (3) according to claim 1,
wherein the estimation unit (3B) estimates whether or not the viewpoint of the endoscope (2) that has captured the endoscopic image (4) is trackable, based on a classification result of classifying the endoscopic image (4) into any of a plurality of classes including a trackable class and an untrackable class.

4. The information processing apparatus (3) according to claim 1,
wherein the estimation unit (3B) estimates whether or not the viewpoint of the endoscope (2) that has captured the endoscopic image (4) is trackable, using a type of an unknown structure region that is included in the endoscopic image (4) and that is determined in advance to affect the tracking of the viewpoint of the endoscope (2).

5. The information processing apparatus (3) according to claim 1,
wherein the estimation unit (3B) estimates whether or not the viewpoint of the endoscope (2) that has captured the endoscopic image (4) is trackable, using a range of an unknown structure region that is included in the endoscopic image (4) and that is determined in advance to affect the tracking of the viewpoint of the endoscope (2).

6. The information processing apparatus (3) according to any one of claims 1 to 5,
wherein the estimation unit (3B) estimates whether or not the viewpoint of the endoscope (2) is trackable before the controller (3F) tracks the viewpoint of the endoscope (2).

7. The information processing apparatus (3) according to any one of claims 1 to 5,
wherein the estimation unit (3B) estimates whether or not the viewpoint of the endoscope (2) is trackable after the controller (3F) tracks the viewpoint of the endoscope (2).

8. The information processing apparatus (3) according to any one of claims 1 to 5,
wherein the controller (3F) controls the acquisition unit (3A) such that the acquisition unit (3A) continues to acquire the endoscopic image (4) and controls the estimation unit such that the estimation unit (3B) continues to estimate whether or not the viewpoint of the endoscope (2) is trackable from the endoscopic image (4) acquired by the acquisition unit (3B) even during a period for which the tracking of the viewpoint of the endoscope (2) is temporarily stopped.

9. The information processing apparatus (3) according to claim 8,
wherein the controller (3F) performs control to resume the tracking of the viewpoint of the endoscope (2) in a case where the estimation unit (3B) estimates that the viewpoint of the endoscope (2) is trackable for a new endoscopic image (4) acquired by the acquisition unit (3A) during the period for which the tracking of the viewpoint of the endoscope (2) is temporarily stopped.

10. A storage medium (10B) storing an information processing program (11) executable by a computer (10) to execute a process comprising:
acquiring an endoscopic image (4) of a living body;
estimating whether or not a viewpoint of an endoscope (2) that has captured the endoscopic image (4) is trackable from the acquired endoscopic image (4); and
in a case where it is estimated that the viewpoint of the endoscope (2) is not trackable, temporarily stopping tracking of the viewpoint of the endoscope (2) until the endoscopic image (4), from which it is estimated that the viewpoint of the endoscope (2) is trackable, is acquired.
